# EUROPEAN PATENT SPECIFICATION

(11) **EP 4 404 879 B1**
(45) Date of publication and mention of the grant of the patent: **09.07.2025**
(21) Application number: 22785919.6
(22) Date of filing: 23.09.2022
(51) Int. Cl.: A61F 4/00, G06F 3/01

(54) **NON-INVASIVE TONGUE BASED INTERFACE**
NICHTINVASIVE ZUNGEBASIERTE SCHNITTSTELLE
INTERFACE NON INVASIVE À BASE DE LANGUETTE

(30) Priority: 24.09.2021 DK PA202170463
(43) Date of publication of application: 31.07.2024
(73) Proprietor: Aalborg Universitet, 9220 Aalborg Øst (DK)
(72) Inventor: STRUIJK, Lotte Najanguaq Søvsø Andreasen, 9575 Terndrup (DK); BENTSEN, Bo, 8270 Højbjerg (DK)
(74) Representative: Plougmann Vingtoft a/s
(86) International application number: PCT/DK2022/050196
(87) International publication number: WO 2023/046252

(56) References cited:
- WO-A1-2009/138089
- CA-A1- 2 005 703
- US-A- 5 689 246

## Description

### FIELD OF THE INVENTION

The present invention relates to the field of human-machine interfaces. Especially, the invention relates to the field of tongue based interfaces.

### BACKGROUND OF THE INVENTION

A tongue based sensor or tongue manipulated human-machine interfaces are known and may be preferred, e.g. to allow disabled persons to control a computer or other device, e.g. a motorized wheelchair. However, a tongue controlled machine interface has potentially also a variety of alternative applications for both abled and disabled individuals, where the tongue control can be used to supplement or replace traditional hand or finger controlled interfaces, e.g. for controlling a computer game, for controlling of various critical devices requiring the user's arms or hands to be used for other purposes, for controlling drones etc.

An inductive tongue sensor panel is known e.g. from WO2009/138089. A panel with several electric coils at various positions is placed in the oral cavity, especially the palate, and the electric coils can be activated by a metallic activation unit, especially a tongue piercing. By inductive interaction between an electric coil and the activation unit, it is possible to activate a number of different activation zones by means of moving the tongue and thus the tongue piercing. This allows the user to control the inductive tongue sensor panel, thereby allowing the user to control a computer or other electrically controllable device, in principle like a keyboard for sensing pressure by a finger.

However, to manipulate such inductive tongue sensor panel, a tongue piercing is required, or at least the activation unit (e.g. a metal sphere) should be glued onto the user's tongue. Gluing is inconvenient and lasts for less than a day, and both gluing and piercing may involve a health risk - the user may swallow the activation unit in case the glue fails, and the invasive piercing process involves an infection risk. Further, a tongue piercing is generally unacceptable for many potential users. This may especially be seen as a problem for potential users for applications where the user is supposed to use the tongue control interface only in short periods of time.

### SUMMARY OF THE INVENTION

Thus, according to the above description, it is an object of the present invention to provide a reliable non-invasive tongue based interface.

In a first aspect, the invention provides a tongue based interface according to the subject-matter of claim 1.

Such tongue based interface is advantageous, since it eliminates the need for the user to have a metallic activator pierced or glued to the tongue. By means of the guide structure, a non-invasive tongue based interface is provided. The activation unit, e.g. a metal sphere, can via the guide structure be manufactured as an integral part of the sensor panel. An integrated unit for mounting on the user's palate with inductive sensor panel and electronic circuit with wireless communication is already a known product. Thus, in preferred embodiments a one-unit tongue sensor device is provided which can be mounted easily in the oral cavity, and it can be easily removed as well. Thus, this opens up for a variety of alternative applications of tongue interfaces.

The guide structure can be implemented as a simple metal frame structure which is hygienic and easy to implement and to mount fixed to the sensor panel, e.g. in combination with an activation unit in the form of e.g. a metal sphere or ball or half sphere or the like, such as made of titanium, a ferromagnetic material or other metals.

The applications of such tongue based interface is as already known to allow disabled persons, e.g. persons with spinal cord injuries, to control a wheelchair, a robotic manipulator such as an exoskeleton or the like, or simply to allow disabled person to interface a computer as an alternative to a keyboard and/or mouse. However, with the non-invasive tongue based control, the general acceptance of an inductive tongue-computer interface (ITCI) may lead to a variety of new applications of tongue based control for both disabled and able-bodied individuals.

A frame based tongue based interface embodiment has been evaluated by a test with a variety of control tasks compared to a test with a similar sensor panel but with an activation unit glued to the user's tongue. In conclusion, inexperienced users performed well with the novel non-invasive interface, while persons experienced with the invasive interface performed best with the invasive interface.

In the following, preferred features and embodiments will be described.

The guide structure may comprise an element which is mounted fixed in relation to the sensor panel, e.g. to allow the guide structure and thereby also the activation unit to be integrated with the sensor panel. Especially, the guide structure carries the activation unit, e.g. a metal sphere or ball, which thereby preferably forms a one-unit structure together with the sensor panel and the electronic circuit.

In some embodiments, the guide structure comprises a frame with a plurality of elements, and wherein the activation unit is carried by a first element of the frame. Especially, the activation unit is arranged to move along said first element of the frame, such as the first element of the frame penetrating through a through-going hole in the activation unit. Especially, the first element may be movably arranged in at least one dimension in relation to at least one further element of the frame. Specifically, the frame comprises two bars or a rectangle, and wherein the first element is arranged to slide along said bars or two sides of the rectangle, such as the two bars being parallel, and wherein the first and second ends of the first element are connected to respective ones of the two bars. Specifically, the first element comprises a curved element and connected to the two bars so as to allow the activation unit to move in a direction out of said plane. Such embodiments allow a simple metal frame structure to function as guide structure for the activation unit. In some embodiments the first element is connected to pivot in relation to the two parallel bars, e.g. the first element may comprise a straight central portion and end portions, wherein the end portions form a pivot axis around which the first element can pivot in relation the two parallel bars. In some embodiments, the first element is shaped and or connected to the two parallel bars to allow the activation unit to be moved in a plane extended by the two parallel bars, while in other embodiments, the first element may be shaped so that the activation unit to move in a plane parallel with the plane extended by the two parallel bars.

Preferably, the frame structure allows free 2D movement of the activation unit in a plane parallel with the activation zones of the sensor panel, as well as movement perpendicular to that plane, to allow activation of a selected activation zone. This movement of the activation zone out of the 2D plane may be implemented by utilizing gravity. Especially, a frame structure may allow the activation unit to have an initial lower position caused by gravity, and wherein the frame structure allows the activation unit to be pushed upwards towards the sensor panel for activation of an activation zone, by the user's tongue. This may be implemented by a part of the frame that allow bending and/or an element hinged and/or shaped to allow the activation unit to move in a direction out of the 2D plane.

E.g. the frame may be formed by metal elements, such as metal wire or metals bars, with a thickness of 0.5-2.0 mm, such as having a thickness about 1.0 mm. Titanium may be used as a metal, however other metals may be used as well. As an alternative to a metal, the guide structure or frame elements may be formed by a polymer.

In other embodiments, the guide structure comprises a telescopic element connected to the activation unit in one end. Especially, the activation unit may be fixed to one end of the telescopic element, and where the opposite end of the telescopic element has a hinge, e.g. a spherical hinge, which is mounted fixed in relation to the sensor panel. In this way, telescopic movement allows the activation unit to be manipulated for activation of an activation zone of the sensor panel.

In some embodiments, the guide structure is configured for placing the activation unit in a rest or parking position away from the sensor panel, preferably the guide structure is configured for the user's tongue to manipulate the activation unit into the rest or parking position. This may be achieved by structural means of the guide structure, e.g. one or more bends of a frame structure which hinders the activation unit to move when positioned away from the sensor panel, e.g. outside an area of the activation zones, however preferably still the activation unit can be brought back into normal operating position for activation of the activation zones by manipulation of the user's tongue.

Preferably, the tongue sensor device comprises a fastening element arranged to fasten the sensor panel on a position in the user's upper oral cavity. Especially, the fastening element may be arranged to fasten the tongue sensor device to the user's palate or fasten the tongue sensor device to two or more of the user's teeth. The tongue sensor device may be fastened to the user's palate by means of a putty for short term use, and/or by means of a dedicated individually shaped element known from the construction of dental prostheses etc., which may allow the user to wear the tongue sensor device for a longer period of time.

The activation unit is preferably a spherical or half spherical ferrous or metallic element, such as a titanium element. Especially, the spherical element has a diameter of such as 1-8 mm, such as 2-5 mm.

The sensor panel preferably has at least 5 different activation zones, such as 5-10, such as 10-20, such as 10-30 different activation zones. A special sensor panel embodiment can have two sections: one section with 10 activation zones serving as a keypad, and a second section with 8 activation zones serving as a mouse area, i.e. allowing the user to provide a control function known from a computer mouse, e.g. for controlling position of a cursor on a computer display or to steer a physical device. A sensor panel could also have a single zone with at least 5 different activation zones, such as 5-10, such as 10-20, such as 10-30 or a touchpad like function with many different activation zones dedicated to joystick like and button like functions Some embodiments may have one electric coil for each activation zone. However, it is possible to interpolate electric signals from the electric coils related to the activation zones, so that activation can be sensed with a spatial resolution of 1 mm. This allows implementation of activation zones with a size of 1x1 mm implemented with a limited number of electric coils.

The electronic circuit preferably comprises a wireless transmitter arranged to transmit a Radio Frequency (RF) signal indicative of activation of the activation zones of the sensor panel. Hereby, a wired connection to a device external to the user's mouth can be avoided. Such sensor panel and electronic circuit with a wireless RF signal output exists as a commercial product, i.e. a device including a battery and encapsulated to fit to a safe and hygienic operation in the mouth of a user.

In a second aspect, the invention provides a method for tongue based control of the tongue based interface,
- providing a tongue sensor device in the oral cavity of the user, the tongue sensor device comprising a sensor panel comprising a 2D array of a plurality of activation zones and related electric coils,
- carrying, by means of a guide structure, a ferrous or metallic activation unit movably to allow the user's tongue to manipulate the activation unit near one activation zone for activation of said one activation zone, and
- sensing activation of said one activation zone using one or more of the electric coils and generating a wired or wireless output accordingly.

In a third aspect, the invention provides a system comprising
- a tongue based interface according to the first aspect, and
- a device arranged to receive the wired or wireless output from the tongue based interface and to control one or more functions in response to said wired or wireless output.

Especially, the device may be arranged to receive a wireless Radio Frequency output from the tongue based interface. Especially, said device may be a standalone device or a part of another device. E.g. the device may be a general purpose device such as a computer or a smartphone. Especially, the device may be configured to control an assistive device, such as a robotic manipulator, an exoskeleton, a wheelchair or the like. The device may be alternatively or additionally configured for control of a game on a computer or a game console, or the device may be arranged as an additional or alternative computer interface, i.e. with the ability to control various devices, such as vehicles (e.g. a drone) or a dedicated devices (e.g. fire fighter equipment).

Preferably, the device comprises an actuator with a function being controlled in response to the output from the tongue based interface. The device may be such as: an assistive device, a vehicle, a game console, a building part, a door or a port, a ventilation system, a tool, a medical device or part of a medical device, or a robotic device. More specifically, the device may be such as: a wheelchair, an exoskeleton, or an unmanned aerial vehicle, e.g. a drone.

It is appreciated that the described embodiments and features for the mentioned aspects can be intermixed in any way.

### BRIEF DESCRIPTION OF THE FIGURES

The invention will now be described in more detail with regard to the accompanying figures of which
FIG. 1a and 1b illustrates block diagrams of an embodiment in a side view and a top view,
FIG. 2a and 2b illustrate a drawing and a photo of the same embodiment,
FIG. 3 illustrates a drawing another embodiment as well as variants of the guide structure for this embodiment,
FIG. 4 illustrates a drawing of yet another embodiment,
FIG. 5 illustrates a drawing of an embodiment with a telescopic guide structure,
FIG. 6 illustrates a drawing of yet an embodiment, and
FIG. 7 illustrates a block diagram of a system embodiment.

The figures illustrate specific ways of implementing the present invention and are not to be construed as being limiting to other possible embodiments falling within the scope of the attached claim set.

### DETAILED DESCRIPTION OF THE INVENTION

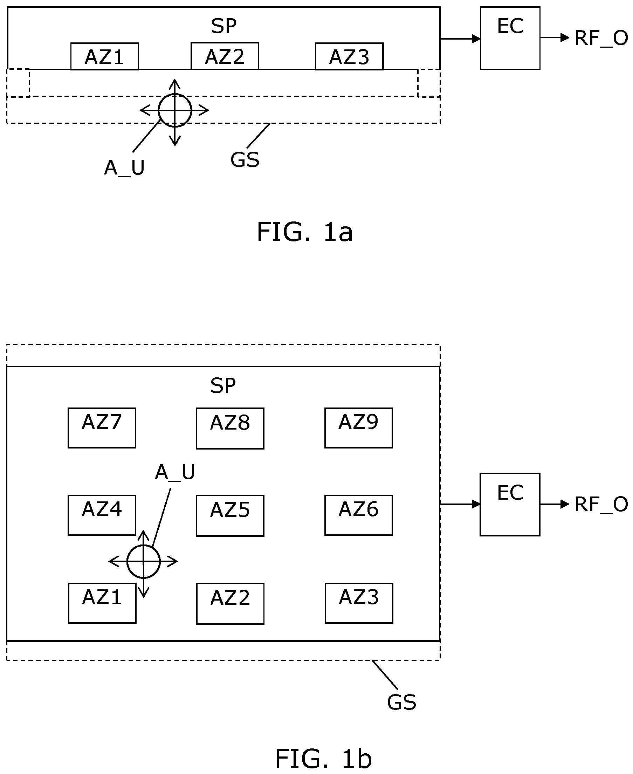

FIG. 1a illustrates a side view of a block diagram form of a tongue based interface embodiment. FIG. 1b illustrates a top view a block diagram from of a tongue based interface of the same embodiment as FIG. 1a. FIG. 1a and 1b illustrate the elements of a tongue sensor device embodiment which is arranged to be fastened on the upper cavity of the user's mouth, so as to allow the user to manipulate the activation unit A_U, preferably a metallic halfsphere, by the user's tongue to activate the desired one of the activation zones AZ1-AZ9 of the sensor panel SP. In this embodiment, the tongue sensor device has a sensor panel SP, guide structure GS, activation unit A_U and electronic circuit EC which are all integrated to form one single unit or one single mouth piece to enter the user's mouth. Thus, the user does not need any invasively tongue mounted activation unit to user the tongue based interface. A tongue based interface with a mouth piece with an inductive sensor panel and electronic circuit with a wireless communication interface is commercially available, and the sensor panel SP and electronic circuit EC according to the invention may be implemented by these already available parts.

The novel feature of the invention is the guide structure GS, preferably mounted to the structure of the sensor panel SP, wherein the guide structure GS carries the activation unit A_U movably in a plane upon manipulation by the user's tongue, preferably a plane parallel with a plane where the activation zones AZ1-AZ9 of the sensor panel SP located, and to allow the activation unit A_U to be moved at a distance from the activation zones AZ1-AZ9. This movability is indicated by the double arrows on the activation unit A_U. This allows the user to move the activation unit A_U to a position in relation to the 2D array of activation zones AZ1-AZ9, and thereby allow the user to position the activation unit A_U near a desired activation zone AZ1-AZ9 to be activated. To activate an activation zone AZ1-AZ9, the guide structure GS allows the activation unit A_U to move in a direction towards the activation zone AZ1-AZ9 by manipulation of the user's tongue, thereby bringing the activation unit A_U so close to one or more coils related to the activation zone AZ1-AZ9 that the electronic circuit EC will detect activation of the activation zone AZ-AZ9. Thus, the guide structure GS will allow 2D movement of the activation unit A_U without activation, and by the user's tongue pressing the activation unit A_U upwards towards the sensor panel, the resulting activation of the electrical coil(s) can be detected by the electronic circuit EC. In preferred embodiments, the electronic circuit EC comprises a Radio Frequency (RF) transmitter arranged to transmit an output in the form of a wireless RF signal RF_O with a representation of data indicating activation of any one of the activation zones AZ1-AZ9 and also indication of which one of the activation zones AZ1-AZ9 which has been activated.

The principle of inductive sensing for detecting activation of an activation zone as well as wireless transmission thereof is known in the art and will thus not be described further. It is to be understood that each activation zone may have its own dedicated electric coil for detection of activation. In other implementations, the number of spatially distributed activation zones may be implemented by less than one coil per activation zone, namely by interpolation between electric signals detected by more than one electric coil. In still other implementations, the number of electric coils is higher than the number of activation zones.

Since the sensor panel SP is arranged for mounting in the upper cavity of the user's mouth, the free 2D movement of the activation unit A_U can be provided by a guide structure GS which carries the activation unit A_U hanging by gravity, i.e. away from the sensor panel SP, and wherein the guide structure GS allows free up and down movement, to allow the user's tongue to lift the activation unit A_U up towards the sensor panel SP for activation of the desired activation zone AZ1-AZ9.

By such guide structure GS arrangement of the activation unit A_U, it is possible to provide the tongue sensor device as one single unit, rather than providing the activation unit A_U as a separate element to be mounted onto the user's tongue.

In the following, various ways of implementing the guide structures GS will be described.

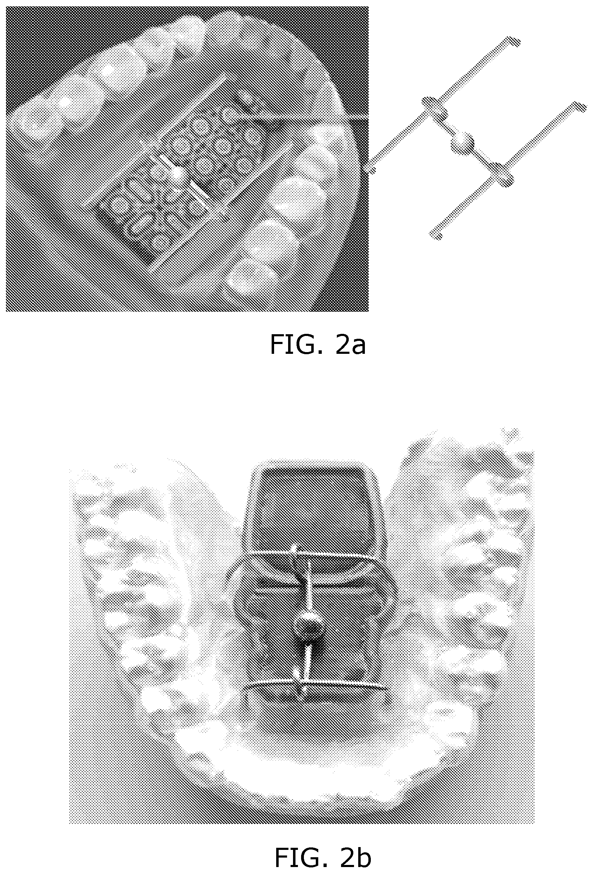

FIG. 2a shows a drawing of an embodiment with a sensor panel with 20 activation zones (electrical coils), and wherein the sensor panel is mounted onto the palate of a user. To the right, the guide structure and activation unit of this embodiment is seen separately for clarity. FIG. 2b shows a photo of a prototype of the same principle for guide structure as in FIG. 2a, but with 18 electrical coils in the sensor panel. The principle of this embodiment is that the guide structure is a frame structure of metal bars or wires that carries the activation unit, here shown as a metal spherical element. The activation unit is arranged to move along a first element of the frame, namely by the first element, a bar element, of the frame penetrating through a through-going hole in the activation unit. The frame structure has two straight parallel bars, and the first element is connected to the respective ones of the two bars by loops or bends, such that the first element can slide along the two bars. Thus, in one direction the activation unit can move along the first element, while in a direction perpendicular thereto, the first element can move along the two straight parallel bars. Via the loops of bends, the activation unit and the first element can hang by gravity, when mounted for normal use. For activation of an activation zone, the user can use the tongue to lift the activation unit upwards, and such upward movement of the activation unit and first element is accommodated by the loop or bend connection to the two bars which are preferably mounted fixed in relation to the sensor panel, such as fixed to a structure of the senor panel.

The prototype shown in FIG. 2b has been tested by experienced as well as not experienced users, and all user managed to perform various tasks requiring activation of different activation zones.

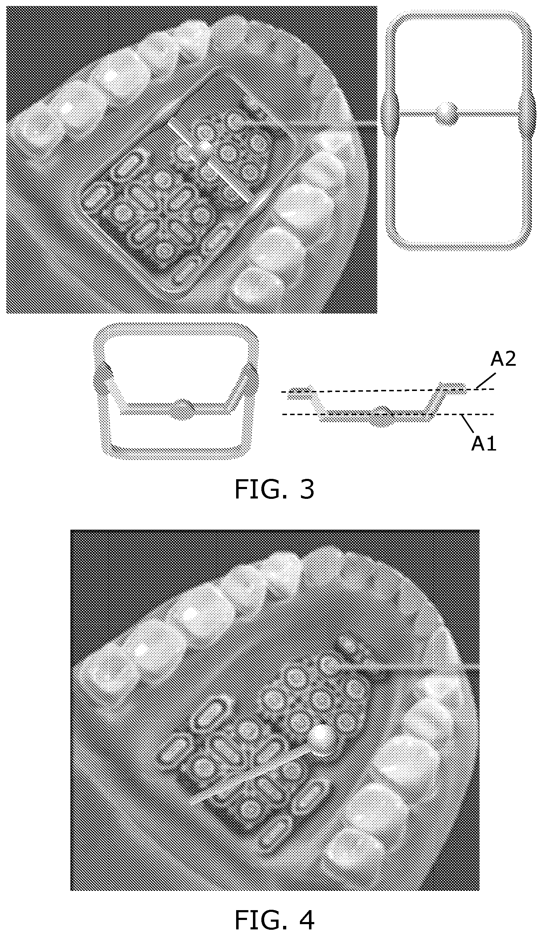

FIG. 3 shows another embodiment, which can be seen as a variant of the one in FIG. 2a and 2b. The where the activation unit is slidably mounted to a cross bar, here shown as a straight bar, wherein the cross bar is slidably connected in both ends to two parallel straight bars. However, in this embodiment the cross bar carrying the activation unit can only slide along the parallel bars, since it is fixed in a direction perpendicular to a plane extended by the two parallel bars. The cross bar and the activation unit can move in the plane extended by the two parallel bars. To allow the activation unit to move towards the sensor panel, for activation, the cross bar and/or the two parallel bars may be made flexible to allow the user's tongue to press the activation unit towards the sensor panel when positioned at the desired position relative to the sensor panel.

The bottom part of FIG. 3 shows an alternative frame structure to the left, where the cross bar is curved. In the shown example, the cross bar has a central straight part, where the activation unit can slide, while its ends are bent, such that the cross bar connects to the two parallel bars such that the activation unit can move in a plane different from the plane extended by the two parallel bars. Hereby, the cross bar may be flexible enough in it its connection to the two parallel bars to allow a bending upon the user's tongue pressing the activation unit against the sensor panel. The bottom part of FIG. 3 to the right shows yet an alternative way of implementing the cross bar, namely where the cross bar has a central straight part forming a first axis A1 along which the activation unit can slide. The ends of the cross bar form a second axis A2, preferably parallel with the first axis A1, and wherein the cross bar is connected by its ends to the two parallel bars to allow the cross bar to pivot around the second axis A2. This allows the activation unit to move perpendicular to the plane extended by the two parallel bars, upon manipulation by the user's tongue, thereby allowing activation of an activation zone on the sensor panel.

FIG. 4 shows an embodiment where the guide structure has a single telescopic straight bar or member with the activation unit mounted fixed on one end, while the other end is mounted fixed in relation to the sensor panel by means of a hinge to allow the activation unit to move.

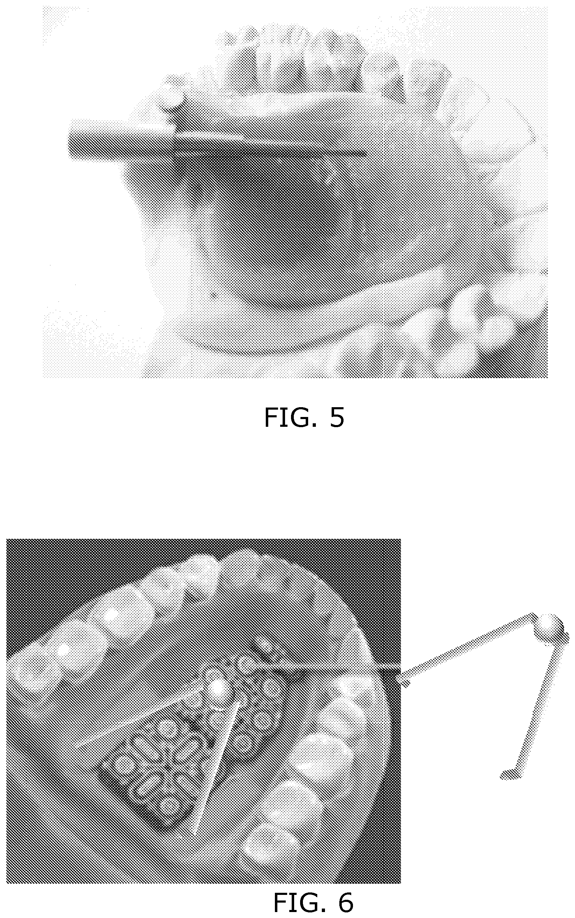

FIG. 5 shows a photo of an embodiment similar to the one in FIG. 4, where the guide structure comprises a telescopic element connected to an activation unit (not shown) at one end, while the opposite end of the telescopic element is mounted to the sensor panel structure so as to at least allow the telescopic element to pivot around an axis perpendicular to the axis of extension of the telescopic element.

FIG. 6 shows an embodiment where the guide structure comprises a double telescopic element where the two telescopic bars are connected at a point where the activation unit is mounted, the other ends of the to two telescopic bars are mounted so as to allow the frame element to pivot and thereby allow the activation unit to move.

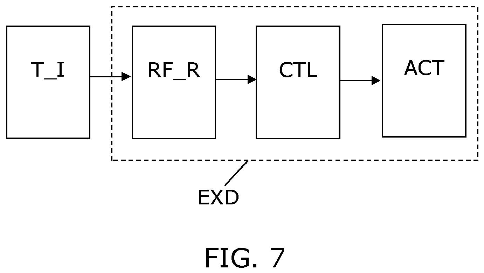

FIG. 7 illustrates a system embodiment with a tongue based interface T_I that provides a wireless RF output to an external device EXD. The external device EXD has a wireless RF receiver RF_R which receives the output from the tongue based interface T_I and provides an output to a controller or computer CTL accordingly. The controller or computer CTL generates in response a control signal for control of an actuator ACT, e.g. an electric motor or linear actuator, an electric switch, a valve or the like, so as to control a function of the external device EXD. E.g. the function to be controlled by the tongue based interface T_I may be such as a moving speed and direction of a vehicle, e.g. a wheelchair.

In an alternative to the embodiment of FIG. 7, the tongue based interface T_I provides a wireless output to an intermediate external device which may perform a processing of the received output, and then transmits a control signal to a target device TD e.g. a robot or computer. The target device then generates a control signal for control of an actuator ACT, e.g. an electric motor or linear actuator, an electric switch, a valve or the like, so as to control a function of the target device TD.

To sum up, the invention provides a non-invasive tongue based interface with a tongue sensor device arranged for position in the oral cavity of the user. A sensor panel to be placed on an upper part of the oral cavity of the user, and having a 2D array of electric coils providing multiple activation zones. A guide structure, e.g. a metal frame, carries a ferrous or metallic activation unit movably in a plane upon manipulation by the user's tongue. This allows the user to position the activation unit in relation the activation zones. The guide structure allows the activation unit to move also perpendicular to the plane, to allow the user's tongue to manipulate the activation unit near said one activation zone for activation of said one activation zone. An electronic circuit senses activation of any one or more of the plurality of activation zones and related electric coils and generates an output accordingly, e.g. a wireless RF output. E.g. a frame structure of metal wires or bars can be used to implement the guide structure, thus allowing the activation unit, e.g. a metal sphere or cylinder, to form an integrated part of the rather than being mounted in an invasive way on the user's tongue.

Although the present invention has been described in connection with the specified embodiments, it should not be construed as being in any way limited to the presented examples. The scope of the present invention is to be interpreted in the light of the accompanying claim set. In the context of the claims, the terms "including" or "includes" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention.

## Claims

1. A tongue based interface (T_I) arranged to receive an input from a user by means of activation by the user's tongue, the tongue based interface comprising
- tongue sensor device arranged for position in the oral cavity of the user, the tongue sensor device comprising
- a sensor panel (SP) comprising a 2D array of a plurality of activation zones (AZ1, AZ2, AZ3) and related electric coils, wherein the sensor panel (SP) is arranged to be positioned in an upper part of the oral cavity of the user,
**characterized in that**
the tongue based interface (T_I) further comprises
- a guide structure (GS) arranged to carry a ferrous or metallic activation unit (A_U) movably in a plane upon manipulation by the user's tongue, so as to allow the user to position the activation unit (A_U) in relation to the 2D array of the plurality of activation zones (AZ1, AZ2, AZ3), and wherein the guide structure (GS) allows the activation unit (A_U) to move in a direction out of said plane in order to allow the user's tongue to manipulate the activation unit (A_U) near one of said activation zones (AZ1, AZ2, AZ3) for activation of said one activation zone (AZ1, AZ2, AZ3), and
- an electronic circuit (EC) connected to the electric coils of the sensor panel (SP) and being arranged to sense activation of one or more of the activation zones (AZ1, AZ2, AZ3) caused by the activation unit (A_U) by means of the related electric coils, and wherein the electronic circuit (EC) is arranged to generate a wireless or wired output (RF_O) accordingly.

2. The tongue based interface (T_I) according to claim 1, wherein the guide structure (GS) comprising an element which is mounted fixed in relation to the sensor panel (SP).

3. The tongue based interface (T_I) according to claim 1 or 2, wherein the guide structure (GS) comprises a frame with a plurality of elements, and wherein the activation unit (A_U) is carried by a first element of the frame.

4. The tongue based interface (T_I) according to claim 3, wherein the activation unit (A_U) is arranged to move along said first element of the frame.

5. The tongue based interface (T_I) according to claim 3 or 4, wherein the first element is movably arranged in at least one dimension in relation to at least one further element of the frame.

6. The tongue based interface (T_I) according to claim 5, wherein the frame comprises two bars, and wherein the first element is arranged to slide along said bars.

7. The tongue based interface (T_I) according to claim 6, wherein the first element comprises a curved element and connected to the two bars so as to allow the activation unit (A_U) to move in a direction out of said plane.

8. The tongue based interface (T_I) according to any of claims 1 or 2, wherein the guide structure (GS) comprises of one or more telescopic elements connected to the activation unit (A_U) in one end.

9. The tongue based interface (T_I) according to any of the preceding claims, wherein the guide structure (GS) is configured for placing the activation unit (A_U) in a rest or parking position away from the sensor panel (SP).

10. The tongue based interface (T_I) according to any of the preceding claims, wherein the tongue sensor device comprises a fastening element arranged to fasten the sensor panel (SP) on a position in the user's upper oral cavity.

11. The tongue based interface (T_I) according to any of the preceding claims, wherein the activation unit (A_U) is a spherical, half-spherical, or cylindrical ferrous or metallic element.

12. The tongue based interface (T_I) according to any of the preceding claims, wherein the electronic circuit (EC) comprises a wireless transmitter arranged to transmit a Radio Frequency signal (RF_O) indicative of activation of the activation zones (AZ1, AZ2, AZ3) of the sensor panel (SP).

13. A method for tongue based control of a tongue based interface, according to claim 1,
- providing a tongue sensor device in the oral cavity of the user, the tongue sensor device comprising a sensor panel (SP) comprising a 2D array of a plurality of activation zones (AZ1, AZ2, AZ3) and related electric coils,
**characterized in that**
the method further comprises
- carrying, by means of a guide structure (GS), a ferrous or metallic activation unit (A_U) movably to allow the user's tongue to manipulate the activation unit (A_U) near one activation zone (AZ1, AZ2, AZ3) for activation of said one activation zone (AZ1, AZ2, AZ3), and
- sensing activation of said one activation zone (AZ1, AZ2, AZ3) using one or more of the electric coils and generating a wired or wireless output (RF_O) accordingly.

14. A system comprising
- a tongue based interface (T_I) according to any of claims 1-12, and
- a device (EXD) arranged to receive the wired or wireless output from the tongue based interface (T_I) and to control one or more functions in response to said wired or wireless output.

15. The system according to claim 14, wherein the device (EXD) is one of:
an assistive device, a vehicle, a game console, a building part, a door or a port, a ventilation system, a tool, a medical device or part of a medical device, or a robotic device.

## Patentansprüche

1. Zungenbasierte Schnittstelle (T_I), die angeordnet ist, um eine Eingabe von einem Benutzer mittels Aktivierung durch die Zunge des Benutzers zu empfangen, wobei die zungenbasierte Schnittstelle Folgendes umfasst:
- Zungensensorvorrichtung, die zur Position in der Mundhöhle des Benutzers angeordnet ist, wobei die Zungensensorvorrichtung Folgendes umfasst:
- eine Sensorplatte (SP), umfassend eine 2D-Anordnung einer Vielzahl von Aktivierungszonen (AZ1, AZ2, AZ3) und zugehörigen elektrischen Spulen, wobei die Sensorplatte (SP) angeordnet ist, um in einem oberen Teil der Mundhöhle des Benutzers positioniert zu werden,
**dadurch gekennzeichnet, dass**
die zungenbasierte Schnittstelle (T_I) ferner Folgendes umfasst:
- eine Führungsstruktur (GS), die angeordnet ist, um eine eisenhaltige oder metallische Aktivierungseinheit (A_U), die in einer Ebene beweglich ist, bei einer Betätigung durch die Zunge des Benutzers zu tragen, um es dem Benutzer zu ermöglichen, die Aktivierungseinheit (A_U) in Bezug auf die 2D-Anordnung der Vielzahl von Aktivierungszonen (AZ1, AZ2, AZ3) zu positionieren, und wobei die Führungsstruktur (GS) es der Aktivierungseinheit (A_U) ermöglicht, sich in einer Richtung aus der Ebene heraus zu bewegen, um es der Zunge des Benutzers zu ermöglichen, die Aktivierungseinheit (A_U) nahe einer der Aktivierungszonen (AZ1, AZ2, AZ3) zur Aktivierung der einen Aktivierungszone (AZ1, AZ2, AZ3) zu betätigen, und
- eine elektronische Schaltung (EC), die mit den elektrischen Spulen der Sensorplatte (SP) verbunden und angeordnet ist, um eine Aktivierung von einer oder mehreren der Aktivierungszonen (AZ1, AZ2, AZ3) zu erfassen, die durch die Aktivierungseinheit (A_U) mittels der zugehörigen elektrischen Spulen verursacht werden, und wobei die elektronische Schaltung (EC) angeordnet ist, um einen drahtlosen oder drahtgebundenen Ausgang (RF_O) entsprechend zu erzeugen.

2. Zungenbasierte Schnittstelle (T_I) nach Anspruch 1, wobei die Führungsstruktur (GS) ein Element umfasst, das in Bezug auf die Sensorplatte (SP) befestigt montiert ist.

3. Zungenbasierte Schnittstelle (T_I) nach Anspruch 1 oder 2, wobei die Führungsstruktur (GS) einen Rahmen mit einer Vielzahl von Elementen umfasst und wobei die Aktivierungseinheit (A_U) durch ein erstes Element des Rahmens getragen wird.

4. Zungenbasierte Schnittstelle (T_I) nach Anspruch 3, wobei die Aktivierungseinheit (A_U) angeordnet ist, um sich entlang des ersten Elements des Rahmens zu bewegen.

5. Zungenbasierte Schnittstelle (T_I) nach Anspruch 3 oder 4, wobei das erste Element in Bezug auf mindestens ein weiteres Element des Rahmens in mindestens einer Dimension beweglich angeordnet ist.

6. Zungenbasierte Schnittstelle (T_I) nach Anspruch 5, wobei der Rahmen zwei Stäbe umfasst und wobei das erste Element angeordnet ist, um entlang der Stäbe zu gleiten.

7. Zungenbasierte Schnittstelle (T_I) nach Anspruch 6, wobei das erste Element ein gebogenes Element umfasst und mit den zwei Stangen verbunden ist, um zu ermöglichen, dass sich die Aktivierungseinheit (A_U) in eine Richtung aus der Ebene heraus bewegt.

8. Zungenbasierte Schnittstelle (T_I) nach einem der Ansprüche 1 oder 2, wobei die Führungsstruktur (GS) ein oder mehrere teleskopische Elemente umfasst, die an einem Ende mit der Aktivierungseinheit (A_U) verbunden sind.

9. Zungenbasierte Schnittstelle (T_I) nach einem der vorhergehenden Ansprüche, wobei die Führungsstruktur (GS) zum Platzieren der Aktivierungseinheit (A_U) in einer Ruhe- oder Parkposition weg von der Sensorplatte (SP) konfiguriert ist.

10. Zungenbasierte Schnittstelle (T_I) nach einem der vorhergehenden Ansprüche, wobei die Zungensensorvorrichtung ein Befestigungselement umfasst, das angeordnet ist, um die Sensorplatte (SP) an einer Position in der oberen Mundhöhle des Benutzers zu befestigen.

11. Zungenbasierte Schnittstelle (T_I) nach einem der vorhergehenden Ansprüche, wobei die Aktivierungseinheit (A_U) ein kugelförmiges, halbkugelförmiges oder zylindrisches eisenhaltiges oder metallisches Element ist.

12. Zungenbasierte Schnittstelle (T_I) nach einem der vorhergehenden Ansprüche, wobei die elektronische Schaltung (EC) einen drahtlosen Sender umfasst, der angeordnet ist, um ein Hochfrequenzsignal (RF_O) zu übertragen, das eine Aktivierung der Aktivierungszonen (AZ1, AZ2, AZ3) der Sensorplatte (SP) angibt.

13. Verfahren zur zungenbasierten Steuerung einer zungenbasierten Schnittstelle nach Anspruch 1,
- Bereitstellen einer Zungensensorvorrichtung in der Mundhöhle des Benutzers, wobei die Zungensensorvorrichtung eine Sensorplatte (SP) umfasst, die eine 2D-Anordnung einer Vielzahl von Aktivierungszonen (AZ1, AZ2, AZ3) und zugehörigen elektrischen Spulen umfasst,
**dadurch gekennzeichnet, dass**
das Verfahren ferner Folgendes umfasst:
- Tragen, mittels einer Führungsstruktur (GS), einer eisenhaltigen oder metallischen Aktivierungseinheit (A_U), die beweglich ist, um der Zunge des Benutzers zu ermöglichen, die Aktivierungseinheit (A_U) nahe einer Aktivierungszone (AZ1, AZ2, AZ3) zur Aktivierung der einen Aktivierungszone (AZ1, AZ2, AZ3) zu betätigen, und
- Erfassen der Aktivierung der einen Aktivierungszone (AZ1, AZ2, AZ3) unter Verwendung einer oder mehrerer der elektrischen Spulen und dementsprechend Generieren einer drahtgebundenen oder drahtlosen Ausgabe (RF_O).

14. System, umfassend:
- eine zungenbasierte Schnittstelle (T_I) nach einem der Ansprüche 1-12 und
- eine Vorrichtung (EXD), die angeordnet ist, um die drahtgebundene oder drahtlose Ausgabe von der zungenbasierten Schnittstelle (T_I) zu empfangen und eine oder mehrere Funktionen als Reaktion auf die drahtgebundene oder drahtlose Ausgabe zu steuern.

15. System nach Anspruch 14, wobei die Vorrichtung (EXD) eines der Folgenden ist:
eine unterstützende Vorrichtung, ein Fahrzeug, eine Spielkonsole, ein Gebäudeteil, eine Tür oder ein Tor, ein Lüftungssystem, ein Werkzeug, eine medizinische Vorrichtung oder ein Teil einer medizinischen Vorrichtung oder eine Robotervorrichtung.

## Revendications

1. Interface basée sur la langue (T_I) conçue pour recevoir une entrée de la part d'un utilisateur au moyen d'une activation par la langue de l'utilisateur, l'interface basée sur la langue comprenant
- un dispositif de capteur de langue conçu pour être positionné dans la cavité buccale de l'utilisateur, le dispositif de capteur de langue comprenant
- un panneau de capteur (SP) comprenant un réseau 2D d'une pluralité de zones d'activation (AZ1, AZ2, AZ3) et des bobines électriques associées, dans lequel le panneau de capteur (SP) est conçu pour être positionné dans une partie supérieure de la cavité buccale de l'utilisateur,
**caractérisée en ce que**
l'interface basée sur la langue (T_I) comprend en outre
- une structure de guidage (GS) conçue pour porter une unité d'activation ferreuse ou métallique (A_U) de manière mobile dans un plan lors d'une manipulation par la langue de l'utilisateur, de façon à permettre à l'utilisateur de positionner l'unité d'activation (A_U) par rapport au réseau 2D de la pluralité de zones d'activation (AZ1, AZ2, AZ3), et dans laquelle la structure de guidage (GS) permet à l'unité d'activation (A_U) de se déplacer dans une direction sortant dudit plan afin de permettre à la langue de l'utilisateur de manipuler l'unité d'activation (A_U) à proximité de l'une desdites zones d'activation (AZ1, AZ2, AZ3) pour l'activation de ladite zone d'activation (AZ1, AZ2, AZ3), et
- un circuit électronique (EC) connecté aux bobines électriques du panneau de capteur (SP) et conçu pour détecter l'activation d'une ou de plusieurs des zones d'activation (AZ1, AZ2, AZ3) provoquée par l'unité d'activation (A_U) au moyen des bobines électriques associées, et dans laquelle le circuit électronique (EC) est conçu pour générer une sortie sans fil ou câblée (RF_O) en conséquence.

2. Interface basée sur la langue (T_I) selon la revendication 1, dans laquelle la structure de guidage (GS) comprenant un élément qui est monté fixe par rapport au panneau de capteur (SP).

3. Interface basée sur la langue (T_I) selon la revendication 1 ou 2, dans laquelle la structure de guidage (GS) comprend un cadre avec une pluralité d'éléments, et dans laquelle l'unité d'activation (A_U) est portée par un premier élément du cadre.

4. Interface basée sur la langue (T_I) selon la revendication 3, dans laquelle l'unité d'activation (A_U) est conçue pour se déplacer le long dudit premier élément du cadre.

5. Interface basée sur la langue (T_I) selon la revendication 3 ou 4, dans laquelle le premier élément est agencé de manière mobile dans au moins une dimension par rapport à au moins un autre élément du cadre.

6. Interface basée sur la langue (T_I) selon la revendication 5, dans laquelle le cadre comprend deux barres, et dans laquelle le premier élément est agencé pour glisser le long desdites barres.

7. Interface basée sur la langue (T_I) selon la revendication 6, dans laquelle le premier élément comprend un élément incurvé et connecté aux deux barres de manière à permettre à l'unité d'activation (A_U) de se déplacer dans une direction sortant dudit plan.

8. Interface basée sur la langue (T_I) selon l'une quelconque des revendications 1 ou 2, dans laquelle la structure de guidage (GS) comprend un ou plusieurs éléments télescopiques connectés à l'unité d'activation (A_U) dans une extrémité.

9. Interface basée sur la langue (T_I) selon l'une quelconque des revendications précédentes, dans laquelle la structure de guidage (GS) est configurée pour placer l'unité d'activation (A_U) dans une position de repos ou de stationnement éloignée du panneau de capteur (SP).

10. Interface basée sur la langue (T_I) selon l'une quelconque des revendications précédentes, dans laquelle le dispositif de capteur de langue comprend un élément de fixation conçu pour fixer le panneau de capteur (SP) sur une position dans la cavité buccale supérieure de l'utilisateur.

11. Interface basée sur la langue (T_I) selon l'une quelconque des revendications précédentes, dans laquelle l'unité d'activation (A_U) est un élément ferreux ou métallique sphérique, demi-sphérique ou cylindrique.

12. Interface basée sur la langue (T_I) selon l'une quelconque des revendications précédentes, dans laquelle le circuit électronique (EC) comprend un émetteur sans fil conçu pour transmettre un signal de radiofréquence (RF_O) indicatif de l'activation des zones d'activation (AZ1, AZ2, AZ3) du panneau de capteur (SP).

13. Procédé de commande basé sur la langue d'une interface basée sur la langue, selon la revendication 1,
- fourniture d'un dispositif de capteur de langue dans la cavité buccale de l'utilisateur, le dispositif de capteur de langue comprenant un panneau de capteur (SP) comprenant un réseau 2D d'une pluralité de zones d'activation (AZ1, AZ2, AZ3) et des bobines électriques associées,
**caractérisé en ce que**
le procédé comprend en outre :
- le port, au moyen d'une structure de guidage (GS), d'une unité d'activation ferreuse ou métallique (A_U) de manière mobile pour permettre à la langue de l'utilisateur de manipuler l'unité d'activation (A_U) à proximité d'une zone d'activation (AZ1, AZ2, AZ3) pour l'activation de ladite une zone d'activation (AZ1, AZ2, AZ3), et
- la détection de l'activation de ladite une zone d'activation (AZ1, AZ2, AZ3) en utilisant une ou plusieurs des bobines électriques et en générant une sortie câblée ou sans fil (RF_O) en conséquence.

14. Système comprenant
- une interface basée sur la langue (T_I) selon l'une quelconque des revendications 1 à 12, et
- un dispositif (EXD) conçu pour recevoir la sortie filaire ou sans fil de l'interface basée sur la langue (T_I) et pour commander une ou plusieurs fonctions en réponse à ladite sortie filaire ou sans fil.

15. Système selon la revendication 14, dans lequel le dispositif (EXD) est l'un de : un dispositif d'assistance, un véhicule, une console de jeu, une partie de bâtiment, une porte ou un port, un système de ventilation, un outil, un dispositif médical ou une partie d'un dispositif médical, ou un dispositif robotique.
